# EUROPEAN PATENT APPLICATION

(11) **EP 0 829 269 A1**
(43) Date of publication of application: **18.03.1998**
(21) Application number: 96114554.7
(22) Date of filing: 11.09.1996
(51) Int. Cl.: A61M 25/00, A61M 29/02

(54) **Catheter system**

(71) Applicant: SCHNEIDER (EUROPE) AG, 8180 Bülach (CH)
(72) Inventor: Schwager Michael, 8404 Winterthur (CH)
(74) Representative: Misrachi, Alfred

(57) **Abstract**

The catheter system comprises an elongated catheter shaft 2 with a longitudinal lumen 4 extending therethrough and provided with a closure 6 in the vicinity of the distal end of the shaft. Inside the longitudinal lumen 4 is a removable wire 7 with a tip 14 providing a distally oriented push to closure 6. A balloon 8 is surrounding a portion of the distal area 3 of catheter shaft 2. A guidewire lumen 10 is formed in the distal area 3 of catheter shaft 2 with an entry 11 for the guidewire 12 at the distal end 5 of shaft 2 and an exit 13 for the guidewire 12 which is distal of the distal end 9 of balloon 8. The closure 6 of the longitudinal lumen 4 is distal of the exit 13 of the guidewire lumen 10 whereby the distal tip 14 of wire 7 provides the closure 6 with a push distal of the exit 13 of the guidewire lumen 10.

## Description

This invention relates to a catheter system for percutaneous transluminal insertion into blood vessels, comprising a catheter shaft having proximal and distal areas, a longitudinal lumen extending within the catheter shaft as from the proximal area thereof, a distal obstruction in said longitudinal lumen, independent wire means for insertion into said longitudinal lumen to provide a distally oriented push to said distal obstruction, an inflatable balloon surroundingly mounted on the distal area of the catheter shaft, said balloon having proximal and distal ends, a guidewire lumen formed in the distal area of the catheter shaft with an entry for the guidewire into the catheter shaft at a distal end of the distal area of the catheter shaft and an exit for the guidewire out of the catheter shaft distal of the distal end of the balloon.

A catheter system as described hereinbefore has been shown in the document Acta Radiologica, 57:411-416, of November 1962.

This technology has been a good solution to insert single lumen balloon catheters into blood vessels before the advent of the introducer sheaths as used today. Today, after the advent of the introducer sheaths, this technology provides the smallest outer diameter for balloon catheters with a stiffening wire through the balloon. For such catheters, that technology allows a catheter shaft with only one lumen in the area where the catheter profile already is inevitably increased because of the folded balloon. Furthermore, that system is also of great help in the handling of balloon catheters for balloon exchange purposes, as occurs for example in angioplasty procedures where it is important to keep the guidewire in place in the blood vessel because it avoids the use of relatively long extension wires. And where the catheter has a tendency to kink upon insertion of the catheter in a blood vessel, for example due to flexibility of the catheter shaft at the site of the balloon, the wire means inserted into the longitudinal lumen extending within the catheter shaft help overcoming that tendency by providing additional and controllable stiffness.

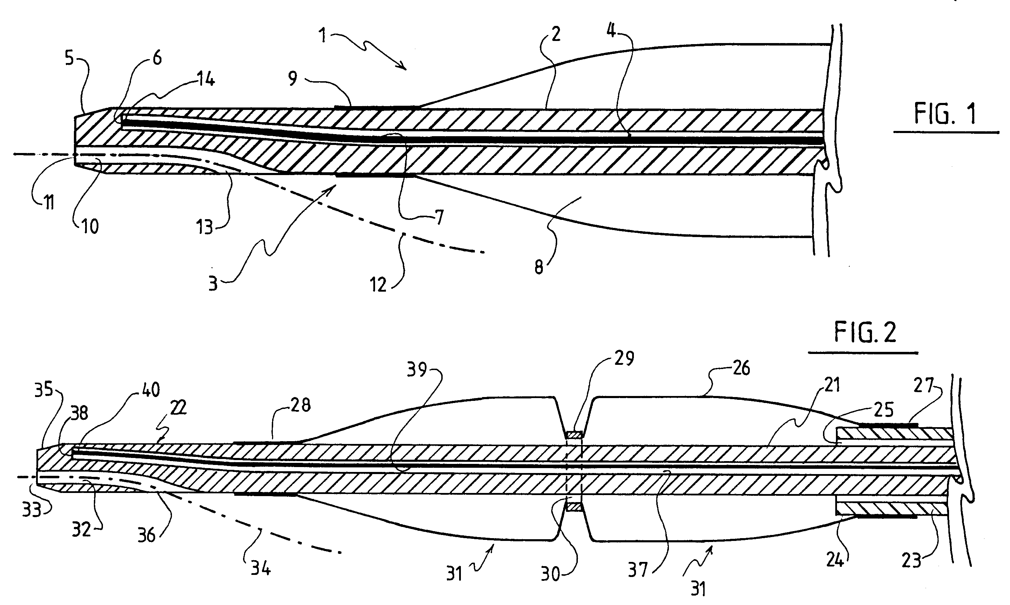

A problem specific to that kind of catheters is that the tip portion of the catheter may go out of way upon insertion and advance of the catheter over the guidewire into a blood vessel thereby clogging upon the guidewire. As may be seen on examples I and II of Fig. 1 of the aforesaid document Acta Radiologica, the distally oriented push force exerted on the catheter shaft by the wire inserted into the longitudinal lumen extending within the catheter shaft is substantially along the longitudinal axis of the catheter shaft, whereas the track reaction at the exit of the guidewire lumen resulting from the friction due to the forward motion of the guidewire lumen over the guidewire is substantially offset to the longitudinal axis of the catheter shaft. As the push force is proximal to the track reaction force, the tip of the catheter shaft tends to rock and stick to the guidewire.

A further problem inherent to those structures, which adds to the rocking tendency of the tip of the catheter shaft and the risk of its going out of way, is that the track reaction resulting from the friction due to the forward motion of the distal end of the guidewire lumen over the guidewire and the force exerted into the longitudinal lumen extending within the catheter shaft are substantially aligned and opposed, whereas this results in an unstable arrangement. As a result, curves or constrictions in the blood vessels may disrupt the initial equilibrium and the tip of the catheter shaft further tends to go out of way and stick to the guidewire.

The document WO 94/03229 is also directed to the general technology of balloon catheter with a guidewire lumen with an entry and an exit for the guidewire distal of the balloon. Within this frame, the document shows a catheter comprising an elongated shaft having a proximal portion defined by a metallic tube. A plastic tube is affixed to a distal end of the metallic tube and a balloon has its proximal end sealingly affixed to the plastic tube. A metallic core member has its proximal end fixedly attached to the metallic tube and extends distally therefrom through the plastic tube and the balloon. The distal end of the balloon is sealingly affixed to the core member the distal portion of which fixedly bears a tip body extending distally of the balloon. The tip body defines a guidewire lumen with an entry and an exit for the guidewire distal of the balloon. The tip body may be formed by a metallic coil or band or tube bonded to the core member, or a plastic tube adhesively secured to the core member. This document does not address the problems of tip portion of the catheter going out of way during insertion and advance of the catheter over the guidewire, as outlined hereinbefore. It does however indicate that where the tip body is formed by a metallic coil, it is contemplated that the windings of the coil are wound in the opposite direction from the windings of the distal portion of the guidewire in order to minimize possible catching or ratcheting of those coils as one component is moved relative to the other. Moreover, since the metallic core member is not removable, the stiffness of the catheter is not controllable as in the document Acta Radiologica. That would certainly not solve the problem of catheter tip going out of way as outlined hereinbefore for catheters of the art described.

US Patent N°5,383,853 also refers to the general technology of balloon catheters with a guidewire lumen with an inlet and an outlet for the guidewire distal of the balloon. And similarly to the material of WO 94/03229, this document relates to a balloon catheter with a tubular member or tip body arranged at the distal end of the catheter shaft and defining a guidewire lumen with inlet and outlet for the guidewire distal of the balloon. The aim is to solve the problem of pinching the guidewire as the catheter is being removed from tortuous paths of blood vessels thereby pulling out the guidewire with the risk of loosing the positioning of the guidewire in the blood vessel. Within this frame, a balloon catheter comprises an elongated shaft in which is located a central core the proximal end of which is fixedly attached to the proximal end of the shaft. The balloon has its proximal end heat shrunk on the catheter shaft and its distal end heat shrunk to the shaft, or to the distal end of the core wire, or over or in the distal tip body or portions thereof, with the core wire inserted therein and glued, bonded or brazed to the distal tip body. The patent also does not address the problems of tip portion of the catheter going out of way during insertion and advance of the catheter upon the guidewire as discussed hereinbefore. By its core and tip body structures the material of the patent substantially assimilates to that of the document WO 94/03229 and the same remarks apply as to the question of solving the problem of catheter tip going out of way during insertion and advance of the catheter upon the guidewire for catheter systems of the art described.

It is an object of this invention to propose a catheter system avoiding the aforesaid drawbacks. A further object of the invention is a catheter system that can be safely operated on a guidewire and moved into tortuous vessels and other extreme conditions. Still a further object of the invention is a catheter system which is versatile and easy to manufacture.

To this effect, the catheter system according to the invention complies with the definitions given in the claims.

Accordingly, where the independent wire means have a distal tip providing push to the distal obstruction distally of the exit of the guidewire lumen, the distally oriented push exerted on the distal obstruction is distal of the track reaction at the exit of the guidewire lumen, which compensates the transverse force resulting from the track reaction at the exit of the guidewire from the guidewire lumen upon advance of the catheter over the guidewire, whereby the rocking tendency of the tip of the catheter shaft is strongly reduced. Furthermore, the distally oriented push provided to the distal obstruction of the longitudinal lumen extending within the catheter shaft and the track reaction resulting from the friction due to the forward motion of the distal end of the guidewire lumen over the guidewire are no more aligned and balancing on each other, the respective lumens overlap, the push and the reaction are turned away from each other. The distal tip of the independent wire means thus trails the catheter shaft and the arrangement of forces at that level becomes stable. The tendency of catheter shaft tip rocking due to disruption of forces is eliminated. The catheter has excellent handling qualities, even through tortuous or narrow vascular configurations. The catheter shaft does not require added tip bodies and the resulting difficulties of assembly. The arrangement of the guidewire lumen distal to the balloon and obstructed longitudinal lumen is place saving and the catheter may have a low profile for better operation into narrow or tortuous blood vessels.

Where the distal tip of the independent wire means provides push to the distal obstruction at a point closer to the distal end of the catheter shaft than to the exit for the guidewire, the compensation of the transverse force resulting from the track reaction at the exit of the guidewire from the guidewire lumen is enhanced, thereby still further reducing the rocking tendency of the tip of the catheter shaft. The trailing effect of the independent wire is amplified and the catheter gets an added stability for best handling into delicate vascular configurations.

The obstruction of the longitudinal lumen may be formed by a constriction of the longitudinal lumen, which allows usage of the longitudinal lumen for injection of fluids into the vascular configuration in addition to its housing function for the push action of the independent wire means.

The obstruction of the longitudinal lumen may also be formed by a closure of the longitudinal lumen which may thus be used for housing a therapy means such as a radioactive radiation source after appropriate positioning of the catheter in the blood vessel and removal of the independent wire means.

Where a balloon catheter is used for positioning a radioactive radiation source into a blood vessel, it is essential for the radioactive radiation source to be centered as exactly as possible inside the vessel in order to avoid the vessel wall being burned. Similar conditions are required for other therapies which have to be evenly distributed to the vessel wall, such as for example heat treatments.

Waisted balloons have been developed to overcome the tendency of balloon catheters with a central lumen to warp on the stretch inside the balloon. However, when a waist is mounted on the balloon, the balloon has a tendency to contract upon inflation, and it is therefore difficult to determine the final length of the balloon when inflated. To overcome this problem, a concentric shaft configuration may be used with the distal end of the balloon sealed to the distal area of the inner shaft and the proximal end of the balloon sealed on the distal area of the outer shaft, whereas the two shafts are not welded to one another as would otherwise be the case but simply held by a luer lock attachment at their proximal ends. This allows proper determination of the assembly but brings in another problem in that shafts which are not welded to one another do not add to each other for stiffness and they are subject to difficulties of pushability because the inner shaft tends to yield or buckle inside the outer shaft and within the balloon upon insertion of the catheter into a blood vessel. Furthermore, buckling of the inner shaft tends to cause an accordion like deformation of the balloon upon insertion of the catheter into the blood vessel. Where such a catheter configuration makes use of a guidewire lumen formed in the distal area of the inner shaft with an entry for the guidewire into the inner shaft at a distal end of the distal area of the inner shaft and an exit for the guidewire out of the inner shaft distal of the distal end of the balloon, the buckling of the inner shaft and deformation of the balloon enhance the risk that the tip portion of the catheter goes out of way upon insertion and advance of the catheter over the guidewire into a blood vessel because they add uncontrollable components to the orientation and equilibrium of the push force in the longitudinal lumen and track reaction forces at the exit and entry of the guidewire lumen as discussed hereinbefore.

Accordingly, where the catheter shaft is formed of an elongated inner shaft having proximal and distal areas, an elongated outer tube surrounding the inner shaft, said outer tube having proximal and distal areas, and inflation lumen means defined between said inner shaft and outer tube, wherein the balloon has its proximal end sealingly surrounding a portion of the distal area of the outer tube and its distal end sealingly surrounding a portion of the distal area of the inner shaft, wherein a waist is mounted on said balloon between the proximal and distal ends thereof, wherein the guidewire lumen is formed in the distal area of the inner shaft with an entry for the guidewire into the inner shaft at a distal end of the distal area of the inner shaft and an exit for the guidewire out of the inner shaft distal of the distal end of the balloon, wherein said longitudinal lumen extends within the inner shaft as from the proximal area thereof with said distal obstruction formed therein, and wherein said independent wire means for insertion into said longitudinal lumen have said distal tip providing push to said distal obstruction of the longitudinal lumen distally of the exit of the guidewire lumen, such an arrangement tensions the inner shaft and the balloon upon insertion and advance of the catheter over the guidewire in a blood vessel, thereby eliminating the aforesaid uncontrolable components. The waisted balloon catheter may be easily pushed through and positioned within tortuous vascular configurations.

These and other objects, features and advantages of the invention will become readily apparent from the following detailed description with reference to the accompanying drawings which show, diagrammatically and by way of example only, preferred but still illustrative embodiments of the invention.

Figure 1 is a longitudinal sectional view of a first embodiment of the catheter system.

Figure 2 is a longitudinal sectional view of a second embodiment of the catheter system.

The catheter system shown in Figure 1 is a balloon catheter 1 comprising an elongated catheter shaft 2 having a distal area 3 and a proximal area (not shown).

A longitudinal lumen 4 extends within the catheter shaft 2 as from its proximal area. Close to the distal end 5 of the shaft 2, the longitudinal lumen 4 is closed at 6.

Inside longitudinal lumen 4, is an independent removable wire 7, for example a stainless steel wire, to provide a distally oriented push to closure 6.

An inflatable balloon 8, for example as used in angioplasty procedures, is surrounding a portion of the distal area 3 of catheter shaft 2; the balloon has its distal end 9 sealingly affixed to the shaft and its proximal end (not shown) also sealingly affixed to the shaft. An inflation lumen (not shown)is provided for along the shaft 2 for supplying inflation fluid to the balloon 8, as commonly practised in the art.

A guidewire lumen 10 is formed in the distal area 3 of the catheter shaft 2, with an entry 11 for the guidewire 12 at the distal end 5 of the shaft 2 and an exit 13 for the guidewire 12 out of the catheter shaft 2 which is distal of the distal end 9 of balloon 8.

As shown in Figure 1, the closure 6 of the longitudinal lumen 4 is distal of the exit 13 of the guidewire lumen 10, whereby the distal tip 14 of the wire 7 provides to the closure 6 a push distal of the exit 13 of the guidewire lumen 10.

Accordingly, when the catheter is inserted into a blood vessel (not shown) and advanced over the guidewire 12, the distally oriented push provided by the tip 14 of wire 7 is distal of track reactions at the exit 13 of the guidewire lumen 10 which may arise from the passage of the guidewire 12 through the guidewire lumen exit 13. And as the push provided by the tip 14 of wire 7 overlaps track reactions at the exit 13 of the guidewire lumen 10 due to the passage of the guidewire 12 therethrough, the tip 4 of wire 7 trails the catheter shaft 2 into the blood vessel.

Although shown as a wire, the independent wire 7 may be made of a coiled steel wire.

The closure 6 may be replaced by other obstructions, for example a constriction of the distal end of the longitudinal lumen or a transverse abutment.

As shown in Figure 1, the closure 6 of the longitudinal lumen 4 is closer to the distal end 5 of the catheter shaft 2 than to the exit 13 of the guidewire lumen 10. Although preferred, this configuration is not compulsory, and the closure 6 of the longitudinal lumen 4 may be positioned otherwise inasmuch as the tip 14 of wire 7 provides to the closure 6 a push distal of the exit 13 of the guidewire lumen 12.

The catheter system shown in Figure 2 comprises a catheter shaft formed of an elongated inner shaft 21 having a proximal area (not shown) and a distal area 22. An elongated outer tube 23 surrounds inner shaft 21, which tube has a proximal area (not shown) and distal area 24. An inflation lumen 25 is defined between the inner shaft 21 and the outer tube 23. The inner shaft 21 and outer tube 23 are not welded to one another as explained hereinbefore.

A balloon 26 has a proximal end 27 sealingly surrounding a portion of the distal area 24 of outer tube 23 and a distal end 28 sealingly surrounding a portion of the distal area 22 of inner shaft 21.

A waist 29 is arranged on the balloon 26 between its ends. The waist 29 may be formed by a molded ring adhesively secured to the balloon, or by a surgical thread, and there may be several waists, as described in Patent Application EP 0688580 A1 which is incorporated hereto by reference. The waist 29 squeezes the balloon 26 to nearly the diameter of the inner shaft 21, thereby leaving a small passage 30 for the inflation fluid injected into the balloon 26 via inflation lumen 25. The waist 29 divides the balloon 26 into similar sections 31 to assure a close center fit of inner shaft 21 within the balloon 26.

A guidewire lumen 32 is formed in the distal area 22 of inner shaft 21, with an entry 33 for the guidewire 34 at the distal end 35 of the distal area 22 of inner shaft 21, and an exit 36 for the guidewire 34 out of the inner shaft 21 which is distal of the distal end 28 of the balloon 26.

A longitudinal lumen 37 extends within inner shaft 21 as from its proximal area (not shown). Close to the distal end 35 of inner shaft 21, the longitudinal lumen 37 is closed at 38.

Inside longitudinal lumen 37 is an independent removable wire 39 to provide a distally oriented push to closure 38. As shown in Figure 2, the closure 38 of longitudinal lumen 37 is distal of the exit 36 of the guidewire lumen 32, whereby the distal tip 40 of the wire 39 provides to the closure 38 a push distal to the exit 36 of guidewire lumen 32.

Accordingly, when the catheter is inserted into a blood vessel (not shown) and advanced over the guidewire 34, the distally oriented push provided by the tip 40 of wire 39 is distal of track reactions at the exit 36 of the guidewire lumen 32 which may arise from the passage of the guidewire 34 through the guidewire lumen exit 36. And as the push provided by the tip 40 overlaps track reactions at the exit 36 of the guidewire lumen 32 due to the passage of the guidewire 34 therethrough, the tip 40 of wire 39 trails the inner shaft 21, and therefore the whole catheter, into the blood vessel, thereby also tensioning the inner shaft 21 and the balloon 26 and avoiding yielding or buckling of the inner shaft 21 and accordion like deformation of the balloon 26.

The configurations of the closure 38 and independent wire 39 as well as the positioning of closure 38 with respect to the exit 36 of the guidewire lumen 32 may be devised as for the embodiment of Figure 1.

When the catheter is adequately positioned in the blood vessel, the wire 39 may be withdrawn from the catheter and replaced by a wire bearing a radioactive radiation source at its end.

## Claims

1. A catheter system for percutaneous transluminal insertion into blood vessels, comprising a catheter shaft (2; 21, 23) having proximal and distal areas (3; 22, 24), a longitudinal lumen (4, 37) extending within the catheter shaft as from the proximal area thereof, a distal obstruction (6, 38) in said longitudinal lumen, independent wire means (7, 39) for insertion into said longitudinal lumen to provide a distally oriented push to said distal obstruction, an inflatable balloon (8, 26) surroundingly mounted on the distal area of the catheter shaft, said balloon having proximal and distal ends (27; 9, 28), a guidewire lumen (10, 32) formed in the distal area of the catheter shaft with an entry (11, 32) for the guidewire (12, 34) into the catheter shaft at a distal end (5, 35) of the distal area of the catheter shaft and an exit (13, 36) for the guidewire out of the catheter shaft distal of the distal end of the balloon, characterized in that said independent wire means (7, 39) have a distal tip (14, 40) providing push to said distal obstruction (6, 38) distally of the exit (13, 36) of the guidewire lumen (10, 32).

2. A catheter system according to claim 1, wherein said distal tip (14, 40) provides push to said distal obstruction (6, 38) at a point closer to the distal end (5, 35) of the catheter shaft (2; 21, 23) than to the exit (12, 36) of the guidewire lumen (10, 32).

3. A catheter system according to claim 1 or 2, wherein said obstruction (6, 38) is formed by a constriction of the longitudinal lumen (4, 37).

4. A catheter system according to 1 or 2, wherein said obstruction (6, 38) is formed by a closure of the longitudinal lumen (4, 37).

5. A catheter system according to any of claims 1 to 4, wherein the catheter shaft is formed of an elongated inner shaft (21) having proximal and distal (22) areas, an elongated outer tube (23) surrounding the inner shaft, said outer tube having proximal and distal (24) areas, and inflation lumen means (25) defined between said inner shaft and outer tube, wherein the balloon (26) has its proximal end (27) sealingly surrounding a portion of the distal area (24) of the outer tube (23) and its distal end (28) sealingly surrounding a portion of the distal area (22) of the inner shaft (21), wherein a waist (29) is mounted on said balloon between the proximal and distal ends (27, 28) thereof, wherein the guidewire lumen (32) is formed in the distal area (22) of the inner shaft (21) with an entry (33) for the guidewire (34) into the inner shaft (21) at a distal end (35) of the distal area (22) of the inner shaft (21) and an exit (36) for the guidewire (34) out of the inner shaft (21) distal of the distal end (28) of the balloon (26), wherein said longitudinal lumen (37) extends within the inner shaft (21) as from the proximal area thereof with said distal obstruction (38) formed therein, and wherein said independent wire means (39) for insertion into said longitudinal lumen (37) have said distal tip (40) providing push to said distal obstruction (38) of the longitudinal lumen (37) distally of the exit (36) of the guidewire lumen (32).
